# EUROPEAN PATENT SPECIFICATION

(11) **EP 1 421 062 B1**
(45) Date of publication and mention of the grant of the patent: **21.11.2007**
(21) Application number: 02758542.1
(22) Date of filing: 16.08.2002
(51) Int. Cl.: C07C 317/20, C07D 213/40, C07C 317/44, C07D 233/56, C07D 211/46, C07D 295/18, C07D 211/60, A61K 31/10, C07D 211/62, A61P 25/28

(54) **NOVEL CYCLOHEXYL SULPHONES**
CYCLOHEXYL-SULPHONE
NOUVELLES CYLOHEXYL SULFONES

(30) Priority: 21.08.2001 GB 0120347; 21.08.2001 WO PCT/GB01/03741
(43) Date of publication of application: 26.05.2004
(73) Proprietor: Merck Sharp & Dohme Limited, Hoddesdon, Hertfordshire EN11 9BU (GB)
(72) Inventor: CHURCHER, Ian, Harlow, Essex CM20 2QR (GB); DINNELL, Kevin, Harlow, Essex CM20 2QR (GB); HARRISON, Timothy, Harlow, Essex CM20 2QR (GB); KERRAD, Sonia, Harlow, Essex CM20 2QR (GB); NADIN, Alan, John, Harlow, Essex CM20 2QR (GB); OAKLEY, Paul, Joseph, Harlow, Essex CM20 2QR (GB); SHAW, Duncan, Edward, Harlow, Essex CM20 2QR (GB); TEALL, Martin, Richard, Harlow, Essex CM20 2QR (GB); WILLIAMS, Brian, John, Harlow, Essex CM20 2QR (GB); WILLIAMS, Susannah, Harlow, Essex CM20 2QR (GB)
(74) Representative: Buchan, Gavin MacNicol
(86) International application number: PCT/GB2002/003806
(87) International publication number: WO 2003/018543

(56) References cited:
- EP-A- 0 863 134
- US-A- 5 703 129

## Description

The present invention relates to a novel class of compounds, their salts, pharmaceutical compositions comprising them, processes for making them and their use in the manufacture of a medicament for treatment of the human body. In particular, the invention relates to novel sulphones which modulate the processing of APP by γ-secretase, and hence are useful in the manufacture of a medicament for treatment or prevention of Alzheimer's disease.

Alzheimer's disease (AD) is the most prevalent form of dementia. Although primarily a disease of the elderly, affecting up to 10% of the population over the age of 65, AD also affects significant numbers of younger patients with a genetic predisposition. It is a neurodegenerative disorder, clinically characterized by progressive loss of memory and cognitive function, and pathologically characterized by the deposition of extracellular proteinaceous plaques in the cortical and associative brain regions of sufferers. These plaques mainly comprise fibrillar aggregates of β-amyloid peptide (Aβ). The role of secretases, including the putative γ-secretase, in the processing of amyloid precursor protein (APP) to form Aβ is well documented in the literature and is reviewed, for example, in WO 01/70677.

There are relatively few reports in the literature of compounds with inhibitory activity towards γ-secretase, as measured in cell-based assays. These are reviewed in WO 01/70677. Many of the relevant compounds are peptides or peptide derivatives.

The present invention provides a novel class of non-peptidic compounds which are useful in the treatment or prevention of AD by modulating the processing of APP by the putative γ-secretase, thus arresting the production of Aβ.

The present invention provides a compound of formula I: 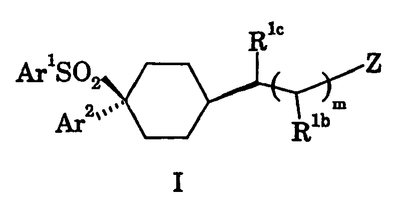 wherein:
m is 0 or 1;
Z represents CN, OR^{2a}, CO₂R^{2a} or CON(R^{2a})₂;
R^{1b} represents H, C₁₋₄alkyl or OH;
R^{1c} represents H or C₁₋₄alkyl;
Ar¹ represents phenyl or pyridyl, either of which bears 0-3 substituents independently selected from halogen, CN, NO₂, CF₃, OH, OCF₃, C₁₋₄alkoxy or C₁₋₄alkyl which optionally bears a substituent selected from halogen, CN, NO₂, CF₃, OH and C₁₋₄alkoxy;
Ar² represents phenyl which is substituted in the 2- and 5-positions with halogen;
R^{2a} represents H, C₁₋₆alkyl, C₃₋₆cycloalkyl, C₃₋₆cycloalkylC₁₋₆alkyl, C₂₋₆alkenyl, any of which optionally bears a substituent selected from halogen, CN, NO₂, CF₃, OR^{2b}, CO₂R^{2b}, N(R^{2b})₂, CON(R^{2b})₂, Ar and COAr; or R^{2a} represents Ar; or two R^{2a} groups together with a nitrogen atom to which they are mutually attached may complete an N-heterocyclyl group bearing 0-4 substituents independently selected from =O, =S, halogen, C₁₋₄ alkyl, CN, NO₂, CF₃, OH, C₁₋₄alkoxy, C₁₋₄alkoxycarbonyl, CO₂H, amino, C₁₋₄alkylamino, di(C₁₋₄alkyl)amino, carbamoyl, Ar and COAr ;
R^{2b} represents H, C₁₋₆alkyl, C₃₋₆cycloalkyl, C₃₋₆cycloalkylC₁₋₆alkyl, C₂₋₆alkenyl, any of which optionally bears a substituent selected from halogen, CN, NO₂, CF₃, OH, C₁₋₄alkoxy, C₁₋₄alkoxycarbonyl, CO₂H, amino, C₁₋₄alkylamino, di(C₁₋₄alkyl)amino, carbamoyl, Ar and COAr; or R^{2b} represents Ar; or two R^{2b} groups together with a nitrogen atom to which they are mutually attached may complete an N-heterocyclyl group bearing 0-4 substituents independently selected from =O, =S, halogen, C₁₋₄alkyl, CN, NO₂, CF₃, OH, C₁₋₄alkoxy, C₁₋₄alkoxycarbonyl, CO₂H, amino, C₁₋₄ alkylamino, di(C₁₋₄alkyl)amino, carbamoyl, Ar and COAr;
Ar represents phenyl or heteroaryl bearing 0-3 substituents selected from halogen, C₁₋₄alkyl, CN, NO₂, CF₃, OH, C₁₋₄alkoxy, C₁₋₄alkoxycarbonyl, amino, C₁₋₄alkylamino, di(C₁₋₄alkyl)amino, carbamoyl, C₁₋₄alkylcarbamoyl and di(C₁₋₄alkyl)carbamoyl;
or a pharmaceutically acceptable salt thereof.

Where a variable occurs more than once in formula I or in a substituent thereof, the individual occurrences of that variable are independent of each other, unless otherwise specified.

As used herein, the expression "C₁₋ₓalkyl" where x is an integer greater than 1 refers to straight-chained and branched alkyl groups wherein the number of constituent carbon atoms is in the range 1 to x. Particular alkyl groups include methyl, ethyl, n-propyl, isopropyl and t-butyl. Derived expressions such as "C₂₋₆alkenyl", "hydroxyC₁₋₆alkyl", "heteroaryl"C₁₋₆alkyl, "C₂₋₆alkynyl" and "C₁₋₆alkoxy" are to be construed in an analogous manner.

The expression "C₃₋₆cycloalkyl" as used herein refers to nonaromatic monocyclic or fused bicyclic hydrocarbon ring systems comprising from 3 to 6 ring atoms. Examples include cyclopropyl, cyclobutyl, cyclopentyl, cyclohexyl, and cyclohexenyl.

The expression "C₃₋₆ cycloalkylC₁₋₆alkyl" as used herein includes cyclopropylmethyl, cyclobutylmethyl, cyclopentylmethyl and cyclohexylmethyl.

The expression "N-heterocyclyl" as used herein means a cyclic or polycyclic system of up to 10 ring atoms selected from C, N, O and S, wherein none of the constituent rings is aromatic and wherein at least one ring atom is nitrogen and bonding is through said ring nitrogen. Preferred N-heterocyclyl groups are monocyclic systems of 4-6 members, such as azetidinyl, pyrrolidinyl, piperidinyl, piperazinyl, morpholinyl, thiomorpholinyl, imidazolidinyl, oxazolidinyl and thiazolidinyl.

The expression "heteroaryl" as used herein means a cyclic or polycyclic system of up to 10 ring atoms selected from C, N, O and S, wherein at least one of the constituent rings is aromatic and comprises at least one ring atom which is other than carbon. Preferred heteroaryl groups are monocyclic systems of 5 or 6 members such as pyridinyl, pyridazinyl, pyrimidinyl, pyrazinyl, pyrrolyl, furyl, thienyl, pyrazolyl, oxazolyl, isoxazolyl, thiazolyl, isothiazolyl, imidazolyl, oxadiazolyl, triazolyl and thiadiazolyl groups. Further examples of heteroaryl groups include tetrazole, 1,2,4-triazine and 1,3,5-triazine.

The term "halogen" as used herein includes fluorine, chlorine, bromine and iodine, of which fluorine and chlorine are preferred.

For use in medicine, the compounds of formula I may advantageously be in the form of pharmaceutically acceptable salts, but other salts may be useful in the preparation of the said compounds or of their pharmaceutically acceptable salts. Suitable pharmaceutically acceptable salts of the compounds of this invention include acid addition salts, such as those formed with hydrochloric, sulphuric, methanesulphonic, fumaric, maleic, succinic, acetic, benzoic, oxalic, citric, tartaric, carbonic or phosphoric acids, and, where the compounds of the invention carry an acidic moiety, sodium, potassium, calcium or magnesium salts, and salts formed with suitable organic ligands, e.g. quaternary ammonium salts or pyridinium salts.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

Regardless of the presence or absence of asymmetric centres, certain compounds in accordance with the invention may exist as enantiomers by virtue of the asymmetry of the molecule as a whole. It is to be understood that in such cases both enantiomers, and mixtures thereof in any proportion, are included within the scope of the invention, and that structural formulae depicting molecules of this type shall be representative of both of the possible enantiomers, unless otherwise indicated.

In the compounds of formula I, Ar¹ represents optionally substituted phenyl or pyridyl, in particular optionally substituted phenyl or 3-pyridyl. Ar¹ is preferably selected from phenyl groups substituted in the 4-position with halogen, methyl or trifluoromethyl and phenyl groups substituted in the 3- and 4-positions by halogen.

Ar² is preferably 2,5-difluorophenyl.

In particular embodiments, Ar¹ is 4-chlorophenyl or 4-trifluoromethylphenyl and Ar² is 2,5-difluorophenyl.

R^{1b} typically represents H, methyl or OH, preferably H.

R^{1c} typically represents H or methyl, preferably H.

When m is 1 R^{1b} and R^{1c} preferably don not both represent C₁₋₄alkyl.

Particular values of R^{2a} include H, phenyl, pyridyl, C₃₋₆cycloalkyl (such as cyclopropyl, cyclobutyl and cyclopentyl), C₃₋₆cycloalkylC₁₋₆alkyl (such as cyclopropylmethyl), C₂₋₆alkenyl (such as allyl), and linear or branched C₁₋₆alkyl which is optionally substituted with CF₃, Ar, OR^{2b}, N(R^{2b})₂, CO₂R^{2b} or CON(R^{2b})₂.

Examples of N-heterocyclyl groups represented by N(R^{2a})₂ include piperidin-1-yl (optionally substituted with OH, CO₂H, CO₂C₁₋₄alkyl, Me or Ph), piperazin-1-yl (optionally substituted with Me or Ph), morpholin-4-yl, thiomorpholin-4-yl, 1,1-dioxo-thiomorphohn-4-yl, 2-oxo-imidazolidin-1-yl, 5,5-dimethyl-2,2-dioxo-oxazolidin-3-yl, 2,5-dioxo-imidazohdin-1-yl, 2-oxo-oxazolidin-3-yl, 2-oxo-pyridin-1-yl, and 2-oxo-pyrrolidin-1-yl.

R^{2b} typically represents H or C₁₋₄alkyl.

When Z represents OR^{2a}, R^{2a} aptly represents H, Ar (especially pyridyl), alkyl (such as methyl, ethyl, propyl or butyl), or substituted alkyl (especially CH₂Ar such as benzyl or pyridylmethyl).

When Z represents CO₂R^{2a}, R^{2a} aptly represents H or alkyl (such as methyl, ethyl, propyl or butyl).

When Z represents CON(R^{2a})₂, the R^{2a} groups independently represent H or optionally substituted alkyl, cycloalkyl, cycloalkylalkyl or alkenyl, or together complete an N-heterocyclyl group. Very aptly, one R^{2a} represents H and the other represents H, alkyl (such as methyl, ethyl, n-propyl, isopropyl, n-butyl, sec-butyl, tert-butyl or 1-ethylpropyl), alkenyl (such as allyl), cycloalkyl (such as cyclopropyl, cyclobutyl or cyclopentyl), cycloalkylalkyl (such as cyclopropylmethyl) or substituted alkyl (such as alkyl substituted with Ar, especially 2-pyridylethyl, 3-(imidazol-1-yl)propyl or 2-phenylethyl; or alkyl substituted with CF₃, CO₂R^{2b}, or CON(R^{2b})₂, especially 2,2,2-trifluoroethyl, methoxycarbonylmethyl or carbamoylmethyl). Alternatively, the two R^{2a} groups complete an N-heterocyclyl group, such as morpholine, thiomorpholine, thiomorpholine-1,1-dioxide, 4-methylpiperazine, 4-phenylpiperazine, piperidine, 4-hydroxypiperidine or piperidine which is substituted in the 3- or 4-position with CO₂R^{2b} and/or C₁₋₄alkyl, especially 3- or 4-carboxypiperidine, 3- or 4-ethoxycarbonylpiperidine, 3-carboxy-3-methylpiperidine and 3-ethoxycarbonyl-3-methylpiperidine.

Examples of individual compounds in accordance with formula I are provided in the Examples section appended hereto.

The compounds of formula I have an activity as modulators of the processing of APP by γ secretase.

The invention also provides pharmaceutical compositions comprising one or more compounds of formula I or the pharmaceutically acceptable salts thereof and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, such as the conventional tableting ingredients known to those skilled in the art, e.g. as described in WO 01/70677, and formed into unit dosage forms. Typical unit dosage forms contain from 0.1 to 500 mg, for example 1, 2, 5, 10, 25, 50, 100 or 200 mg, of the active ingredient. Tablets or pills of the novel composition can be coated or otherwise compounded to provide a dosage form affording the advantage of prolonged action, as described, for example, in WO 01/70677.

The liquid forms in which the novel compositions of the present invention may be incorporated for administration orally or by injection include aqueous solutions, suitably flavoured syrups, aqueous or oil suspensions, and flavoured emulsions with edible oils, as described in WO 01/70677.

The present invention also provides a compound of formula I or a pharmaceutically acceptable salt thereof for use in a method of treatment of the human body. Preferably the treatment is for a condition associated with the deposition of β-amyloid. Preferably the condition is a neurological disease having associated β-amyloid deposition such as Alzheimer's disease.

The present invention further provides the use of a compound of formula I or a pharmaceutically acceptable salt thereof in the manufacture of a medicament for treating or preventing Alzheimer's disease.

Also disclosed is a method of treatment of a subject suffering from or prone to Alzheimer's disease which comprises administering to that subject an effective amount of a compound according to formula I or a pharmaceutically acceptable salt thereof.

For treating or preventing Alzheimer's disease, a suitable dosage level is about 0.01 to 250 mg/kg per day, preferably about 0.05 to 100 mg/kg per day, and especially about 0.1 to 50 mg/kg of body weight per day. The compounds may be administered on a regimen of 1 to 4 times per day. In some cases, however, dosage outside these limits may be used.

Compounds of formula I in which m is 0 and Z is CO₂R^{2a} or CON(R^{2a})₂ may be prepared by coupling of a carboxylic acid (1) with (respectively) R^{2a}OH, or HN(R^{2a})₂, 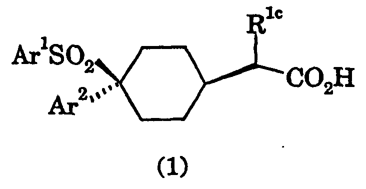 where Ar¹, Ar², R^{1c} and R^{2a} have the same meanings as before. Any of the standard coupling techniques may be used, including the use of coupling agents such as dimethylaminopyridine, hydroxybenzotriazole, dicyclohexylcarbodiimide, carbonyldiimidazole and the like. In one preferred method, the acid is converted to the corresponding acid chloride (e.g. by treatment with oxalyl chloride in DMF solution) and reacted directly with the desired nucleophile. In another preferred method, the acid is converted to an active ester derivative such as the pentafluorophenol ester (e.g. by coupling with the phenol in the presence of dicyclohexyl carbodiimide), and this intermediate is reacted with the desired nucleophile.

The acids (1) are available by hydrolysis of the esters (2), typically under alkaline conditions such as treatment with LiOH in ethanol solution: 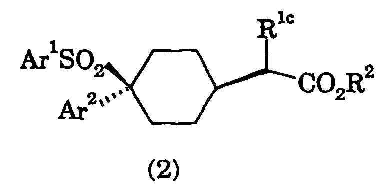 where R² represents alkyl such as methyl or ethyl, and Ar¹, Ar² and R^{1c} have the same meanings as before.

The esters (2) are available by reduction of the alkylidene derivatives (3), optionally followed by alkylation with (C₁₋₄alkyl)-L where L is a leaving group (especially bromide or iodide) when R^{1c} is other than H: 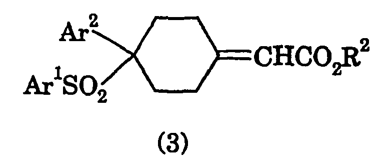 where Ar¹, Ar² and R² have the same meanings as before. The reduction may be carried out using sodium borohydride and nickel(II) chloride in ethanol, while the optional alkylation may be effected by treating the ester (2, R^{1c} = H)) with strong base (e.g. sodium bis(trimethylsilyl)amide) in an aprotic solvent at low temperature, followed by treatment with (C₁₋₄alkyl)-L and warming to room temperature.

If desired, the unsaturated esters (3) may be hydrolysed to the corresponding acids and converted to amides by reaction with HN(R^{2a})₂ prior to reduction of the olefinic bond.

The unsaturated esters (3) are available from condensation of a ketone (4) with Ph₃P=CHCO₂R²: 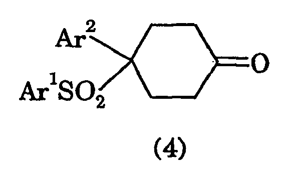 where Ar¹, Ar² and R² have the same meanings as before, while the ketones (4) are available by decarboxylation of the enols (5), which in turn are formed by reaction of a sulphone (6) with at least two equivalents of an acrylate (7): 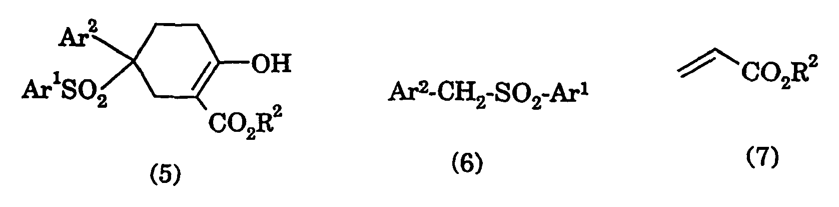 where Ar¹, Ar² and R² have the same meanings as before. The decarboxylation may be accomplished by heating at 150°C in DMSO in the presence of sodium chloride and water, while reaction of (6) with (7) may be carried out at ambient temperature in an inert solvent such as THF in the presence of strong base such as potassium t-butoxide.

The sulphones (6) are prepared by oxidation of thioethers Ar²-CH₂-SAr¹ (8), which in turn are formed by reaction of thiols Ar¹SH (9) with benzyl derivatives Ar²CH₂-L (10), where L is a leaving group such as chloride or bromide and Ar¹ and Ar² have the same meanings as before. The reaction between (9) and (10) takes place in an inert solvent such as dichloromethane in the presence of a base such as triethylamine, while the oxidation of (8) to (6) is conveniently effected by m-chloroperoxybenzoic acid, also in an inert solvent such as dichloromethane.

Compounds of formula I in which m is 0 and Z is CN or OR^{2a} may be obtained by reaction of a sulphonate ester (11) with (respectively) cyanide ion or R^{2a}OH: 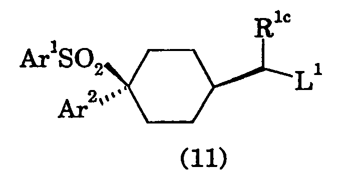 where L¹ represents a sulphonate leaving group (such as mesylate, tosylate or triflate) and Ar¹, Ar², R^{1c} and R^{2a} have the same meanings as before. The displacement reaction may be carried out in DMF at elevated temperature, e.g. about 80°C. When the nucleophile is R^{2a}OH, it is advantageous to generate the corresponding anion by treatment with sodium hydride prior to reaction with (11).

The sulphonates (11) are prepared by reaction of the alcohols (12) with the appropriate sulphonyl chloride (e.g. under anhydrous conditions at low temperature in the presence of a tertiary amine).

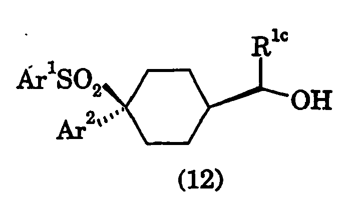

The alcohols (12) are available from the hydroboration of alkenes (13): 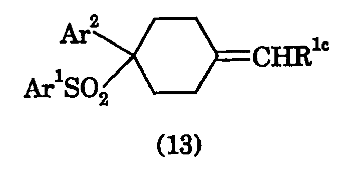 wherein Ar¹, Ar² and R^{1c} have the same meanings as before. The process typically involves reaction with borane in THF at room temperature, followed by treatment with alkaline hydrogen peroxide and separation of the desired *cis* isomer by chromatography. Alkenes (13) are available from ketones (4) by condensation with Ph₃P=CHR^{1c} where R^{1c} has the same meaning as before.

An alternative route to the alcohols (12) in which R^{1c} is H involves reduction of a ketone (4) (e.g. using borohydride) to the corresponding secondary alcohol (14), converting said alcohol (14) to the corresponding mesylate (or equivalent leaving group), effecting nucleophilic displacement with cyanide ion, hydrolysing the resulting nitrile to the corresponding carboxylic acid, followed by reduction to the primary alcohol. The hydrolysis is typically carried out under acid conditions (e.g. in a mixture of acetic acid and conc. HCl at 110°C) and the reduction is conveniently carried out by sequential treatment with isobutyl chloroformate and borohydride in THF.

Compounds of formula I in which m is 1 and R^{1b} is H or C₁₋₄alkyl may be obtained via oxidation of an alcohol (12) to the corresponding aldehyde or ketone, and elaboration of the carbonyl group thereof in the manner described previously in connection with conversion of the ketones (4) into compounds of formula I in which m is 0. For example, oxidation of the alcohols (12) wherein R^{1c} is H provides the corresponding cyclohexanecarboxaldehydes, which may be condensed with Ph₃P=CO₂Et to provide ethyl esters of 2-cyclohexylpropenoic acids. These in turn may be hydrogenated to the corresponding ethyl 2-cyclohexylpropanoates, which optionally may be subjected to alkylation, and/or to hydrolysis to the corresponding acids, and/or converted to a variety of amide or alternative ester derivatives.

Where they are not themselves commercially available, the starting materials and reagents employed in the above-described synthetic schemes may be obtained by the application of standard techniques of organic synthesis to commercially available materials.

It will be appreciated that many of the above-described synthetic schemes may give rise to mixtures of stereoisomers. In particular, certain products may be formed as mixtures of *cis* and *trans* isomers in which a particular ring substituent is on the same or opposite side of the ring as the arylsulphonyl group. Such mixtures may be separated by conventional means such as fractional crystallisation and preparative chromatography.

Certain compounds according to the invention may exist as optical isomers due to the presence of one or more chiral centres or because of the overall asymmetery of the molecule. Such compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as (-)-di-p-toluoyl-d-tartaric acid and/or (+)-di-p-toluoyl-1-tartaric acid, followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

Suitable methods of assaying the level of activity of compounds of the present invention towards γ-secretase are disclosed in WO 01/70677 and in Biochemistry, 2000, 39(30), 8698-8704.

The Examples of the present invention all had an ED₅₀ of less than 10µM, preferably less than 1µM and most preferably less than 100nM in at least one of the above-referenced assays.

The following examples illustrate the present invention.

### EXAMPLES

### Intermediate 1

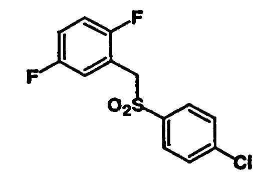

4-Chlorothiophenol (3.6g, 0.025mol) in dichloromethane (100ml) was treated with 2,5-difluorobenzyl bromide (5.17g, 0.025mol) and triethylamine (3.9ml, 0.028mol), reaction was stirred for 2 hours then diluted with dichloromethane (250ml) and washed with water (100ml) and brine (100ml). The separated organic layer was dried (MgSO₄) and evaporated to dryness. Product was purified by passing down a plug of silica eluting with hexane-ethyl acetate mixtures. 5.12g. ¹H NMR CDCl₃ 7.23 (4H,s), 6.69-6.86 (3H,m) and 4.04 (2H,s).

This thioether (5.12g, 0.018mol) was dissolved in dichloromethane (100ml) and treated with m-chloroperoxybenzoic acid (14.3g, 0.042mol (50%w/w)) and stirred for 2 hours. The reaction was then washed with Na₂S₂O₅ (5% solution, 100ml), brine (50ml), dried (MgSO₄) and evaporated to dryness. The sulphone product was purified on silica eluting with hexane-ethyl acetate mixtures, 3.6g. ¹H NMR CDCl₃ 7.61 (2H,d, J=8.6Hz), 7.45 (2H,d, J=8.6Hz), 7.13-7.08 (1H,m), 7.05-7.01 (1H,m), 7.05-7.00 (1H,m), 6.99-6.87 (1H,m) and 4.36 (2h,s).

### Intermediate 2

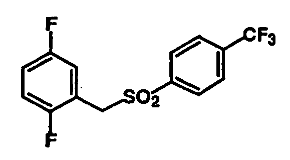

Prepared as for Intermediate 1, using 4-trifluoromethylthiophenol, and obtained as a solid. ¹H NMR (360MHz, CDCl₃) δ 7.85-7.83 (2H, m), 7.76-7.74 (2H, m), 7.15-7.10 (1H, m), 7.06-7.0 (1H, m), 6.92-6.86 (1H, m) and 4.46 (2H, s).

### Preparation 1

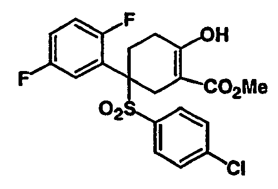

Intermediate 1 (1g, 3.31mmol) and methyl acrylate (0.84ml, 9.27mmol) in tetrahydrofuran (30ml) were treated dropwise with potassium ^{t}butoxide (3.64ml 1M solution in tetrahydrofuran, 3.64mmol). The reaction was stirred for 2 hours, diluted with ethyl acetate (100ml) and washed with water (50ml) and brine (50ml). The organic phase was separated, dried (MgSO₄) and evaporated to dryness, and the product purified on silica eluting with hexane-ethyl acetate mixtures.(1.0g). ¹H NMR CDCl₃ 12.0 (1H,s), 7.41 (4H,s), 7.06-7.0 (2H,m), 6.87-6.81 (1H,s), 3.81 (3H,s), 3.38 (1H,dd, J=3.2,15.8Hz), 3.02-2.92 (2H,m), 2.52 (1H,dd, J= 5.7, 18.5Hz), 2.3-2.2 (1H,m) and 2.2-2.1 (1H,m).

### Preparation 2

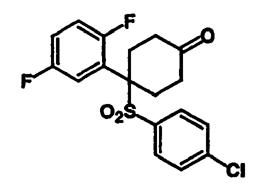

The ester from Preparation1 (1.0g,2.25mmol) in dimethylsulfoxide (10ml) was treated with NaCl (0.3g, 4.96mmol) and water (0.9ml, 4.96mmol) and heated at 150°C for 2 hours. The cooled reaction mixture was diluted with ethyl acetate (100ml), washed with saturated NH₄Cl (100ml), and the organic phase separated, dried (MgSO₄) and evaporated to dryness. The product was purified on silica eluting with hexane-ethyl acetate mixtures, 0.5g. ¹H NMR CDCl₃ 7.43-7.37 (4H,m), 7.22-7.1 (2H,m), 6.97-6.9 (1H,m), 3.05-2.98 (2H,m) and 2.61-2.53 (2H,m).

### Preparation 3

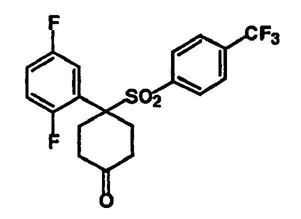

Prepared by the procedures of Preparations 1 and 2 using Intermediate 2 to give the product as a solid. (0.3g) ¹H NMR (360MHz, CDCl₃) δ 7.71-7.69 (2H, d, J= 7.5Hz), 6.62-6.60 (2H, d, J= 7.4Hz), 7.22-7.11 (2H, m), 6.95-6.88 (1H, m), 3.02-2.99 (2H, m), 2.63-2.54 (4H, m) and 2.25-2.16 (2H, m).

### Preparation 4

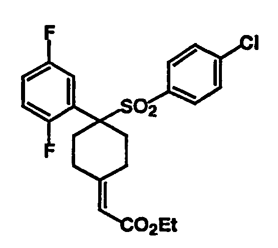

Ethyl (diethoxyphosphinyl)acetate (5.16 mL, 26 mmol) was added dropwise to a slurry of sodium hydride (60% dispersion in mineral oil, 988 mg, 24.7 mmol) in tetrahydrofuran (60 mL) and the mixture was stirred at room temperature for 1 h. The ketone from Preparation 2 (5 g, 13 mmol) in tetrahydrofuran (50 mL) was added dropwise over 20 min. and the mixture was stirred at room temperature for 18 h. Water was added and the mixture was extracted with ethyl acetate. The combined organic fractions were washed with water, dried (MgSO₄ and the solvent was evaporated under reduced pressure. The residue was purified by flash column chromatography on silica gel, eluting with isohexane:EtOAc (85:15), to give the product as a white solid (5.2 g, 88%). ¹H NMR (400MHz, CDCl₃) δ 7.41-7.36 (4H, m), 7.18-7.13 (1H, m), 7.11-7.05 (1H, m), 6.93-6.86 (1H, m), 5.64 (1H, s), 4.14-4.10 (2H, m), 3.99-3.96 (1H, m), 2.91-2.80 (2H, m), 2.42-2.38 (1H, m), 2.31-2.04 (3H, m), 1.89-1.78 (1H, m), 1.28-1.24 (3H, m).

### Preparation 5

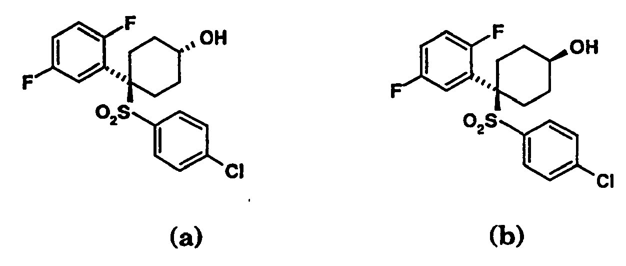

The ketone from Preparation 2, (0.1g,0.26mmol) in methanol (2ml) was treated with NaBH₄ (0.098g, 0.26mmol) and stirred for 1 hour. The reaction was quenched with HCl (1N, 10ml), diluted with ethyl acetate (20ml), then the organic phase was separated, dried (MgSO₄) and evaporated to dryness. The *cis* and *trans* products were purified on silica eluting with hexane-ethyl acetate mixtures.
**(a)** (*trans*) 0.052g. ¹H NMR CDCl₃ 7.39-7.33 (4H,m), 7.11-7.02 (2H,m), 6.88-6.82 (1H,m), 3.80-3.73 (1H,m), 2.80-2.60 (2H,m), 2.22-2.16 (2H,m), 2.08-2.04 (2H,m), 1.53(1H,br) and 1.27-1.13 (2H,m).
**(b)** (*cis*) ¹H NMR (CDCl₃) 7.40 (4H,s), 7.16-7.03 (2H,m), 6.90-6.83 (1H,m), 3.97-3.95 (1H, m), 3.77-3.68 (1H, m), 3.51-3.49 (1H, m), 2.61-2.53 (2H,m), 1.91-1.83 (2H, m) and 1.50-1.42 (2H, m).

### Preparation 6

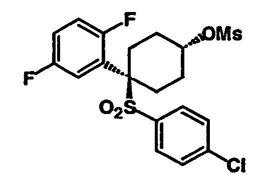

The *trans* cyclohexanol from Preparation 5 (2.7g, 6.9mmol) and triethylamine (1.45mL, 10.3mmol) in dichloromethane (50mL) were treated with methane sulphonyl chloride (0.645mL, 8.9mmol) at -30°C. After 30 mins the mixture was washed with water (20mL), 10% aqueous citric acid (20mL) and saturated aqueous sodium hydrogen carbonate (50mL), dried (MgSO₄ and evaporated to dryness. The solid was triturated with ether to give the mesylate (2.6g) ¹H NMR (CDCl₃) 7.40-7.37 (4H,m), 7.12-7.07 (2H,m), 6.92-6.83 (1H,m), 4.78-4.65 (1H, m), 2.96 (3H, s), 2.88-2.52 (2H, m), 2.29-2.21 (4H, m) and 1.59-1.47 (2H, m).

### Preparation 7

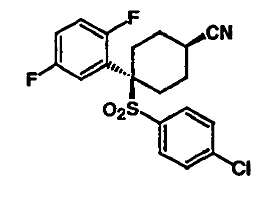

The *trans* mesylate from Preparation 6 (103mg, 0.22mmol) was dissolved in toluene (20ml) and added to a pre-azeotroped sample of tetrabutylammonium cyanide (354mg, 1.32mmol). and the mixture was warmed to 70°C over 18hr and then cooled to rt. The solution was diluted with water (10ml) and washed with ethyl acetate (2 x 50ml). The organic phase was washed with brine (10ml), dried (MgSO₄) and evaporated. The clear oil obtained was purified by column chromatography on silica gel eluting with 10-20% ethyl acetate in hexanes, to give the cyanide. ¹H NMR (CDCl₃) 7.42-7.36 (4H, s), 7.10-7.05 (2H, m), 6.89-6.84 (1H, m), 2.88-2.86 (1H, m), 2.76-2.72 (2H, m), 2.52-2.45 (1H,m), 2.12-2.07 (1H, m) and 1.56-1.49 (1H, m).

### Preparation 8

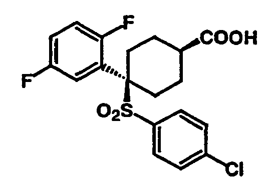

The cyanide from Preparation 7 (143mg, 0.36mmol) was dissolved/suspended in a mixture of glacial acetic acid (10ml) and conc. HCl (6ml) and heated at 110°C for 15 hours. The mixture was cooled, diluted with ethyl acetate and washed with water (x3), dried (MgSO₄) and evaporated to dryness. This crude residue (153mg) was purified by preparative tlc (5% methanol in dichloromethane/ 1% acetic acid). ¹H NMR (CDCl₃) 7.38-7.35 (4H, s), 7.08-7.06 (2H, m), 6.90-6.84 (1H, m), 2.65-2.58 (2H, m), 2.38-2.33 (3H, m), and 1.75-1.49 (4H, m).

### Preparation 9

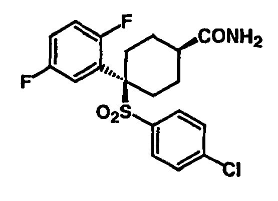

The cyanide from Preparation 8 (50mg, 0.12mmol) was dissolved in a mixture of tetrahydrofuran (4.5ml) and water (0.5ml) and stirred at 20°C. The mixture was treated with hydrogen peroxide (20ml, 0.6mmol) and then with lithium hydroxide (6mg, 0.25mmol) for 2 hours. Hydrogen peroxide (20ml, 0.6mmol) and then with lithium hydroxide (6mg, 0.25mmol) were added and the mixture was stirred at rt. for 72hrs. The mixture was cooled, diluted with ethyl acetate and washed with water (x2) and sat. sodium bisulphite, dried (MgSO₄) and evaporated to dryness. This crude residue (51mg) was purified by preparative tlc (20% ethyl acetate in hexanes) ¹H NMR (CDCl₃) 7.37 (4H, s), 7.10-7.02 (2H, m), 6.90-6.84 (1H, m), 5.57 (2H, brs), 2.54-2.48 (3H, m), 2.43-2.39 (1H, m), 2.19-2.15 (2H, m) and 1.62-1.50 (3H, m).

### Example 1

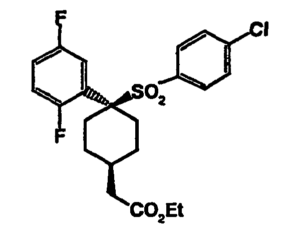

Sodium borohydride (313 mg, 8.23 mmol) was added to a mixture of the unsaturated ester from Preparation 4 (3.74 g, 8.23 mmol) and nickel (II) chloride (2.67 g, 20.6 mmol) in ethanol (100 mL). The mixture was stirred at room temperature for 20 min., then water (100 mL) was added. The mixture was filtered through Hyflo^{™}, washing with ethanol and ethyl acetate. The solvent was evaporated under reduced pressure and the residue was partitioned between ethyl acetate and water. The organic layer was collected, dried (MgSO₄) and the solvent was evaporated under reduced pressure. The residue was purified by flash column chromatography on silica gel, eluting with isohexane:EtOAc (85:15), to give the faster running *cis*-isomer, as an oil (1.36 g, 36%), ¹H NMR (400MHz, CDCl₃) δ 7.37-7.30 (4H, m), 7.09-7.00 (2H, m), 6.86-6.79 (1H, m), 4.14 (2H, q, J=7.1 Hz), 2.47 (2H, d, J=7.6 Hz), 2.46-2.38 (2H, m), 2.19-2.14 (1H, m), 1.76-1.71 (2H, m), 1.57-1.48 (4H, m), 1.27 (3H, t, J 7.1 Hz);
and also the slower running *trans*-isomer, as an oil (200 mg, 5.3%).

### Example 2

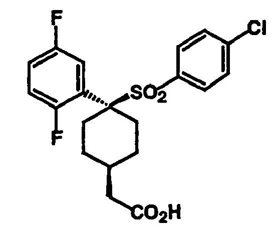

Lithium hydroxide (350 mg, 14.57 mmol) was added to a solution of the cis-ester from Example 1, (1.33 g, 2.91 mmol) in ethanol (40 mL). The mixture was degassed and stirred at room temperature under nitrogen gas for 5 h. The mixture was poured into aqueous hydrochloric acid (1M) and extracted with ethyl acetate. The organic extract was dried (MgSO₄) and the solvent was evaporated under reduced pressure to give a white solid which was then crystallized from IPA to give the product as a white solid (950 mg, 76%). ¹H NMR (400MHz, CD₃OD) δ 7.51-7.49 (2H, m), 7.40-7.37 (2H, m), 7.19-7.10 (2H, m), 7.00-6.94 (1H, m), 2.51-2.35 (6H, m), 2.13-2.10 (1H, m), 1.78-1.74 (2H, m), 1.57-1.50 (2H, m).

### Example 3

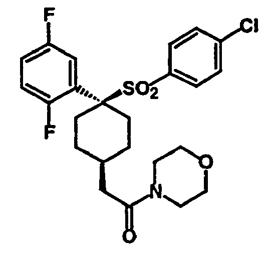

The acid from Example 2 (50 mg, 0.117 mmol), morpholine (30 µL, 0.351 mmol), 1-hydroxybenzotriazole (24 mg, 0.176 mmol) and triethylamine (65 µL, 0.468 mmol) was stirred in tetrahydrofuran at room temperature under nitrogen gas for 10min. 1-(3-Dimethylaminopropyl)-3-ethylcarbodiimide hydrochloride (45 mg, 0.234 mmol) was added to the mixture and stirred for 24 h. The mixture was poured into aqueous sodium hydroxide (1M) and extracted with ethyl acetate. The organic extract was dried (MgSO₄) and the solvent was evaporated under reduced pressure. The residue was purified by flash column chromatography on silica gel, eluting with 5 to 10% methanol in dichloromethane, to give the product as a white foam (50 mg, 86%). ¹H NMR (400MHz, CD₃OD) δ 7.50 (2H, d, *J* 8.6 Hz), 7.37 (2H, d, *J* 8.6 Hz), 7.19-7.09 (2H, m), 7.00-6.93 (1H, m), 3.69-3.63 (4H, m), 3.59-3.56 (4H, m), 2.55 (2H, d, *J* 7.4 Hz), 2.47-2.39 (4H, m), 2.16-2.07 (1H, m), 1.78-1.74 (2H, m), 1.58-1.51 (2H, m). m/z (ES⁺) (M+1) 498 + 500.

### Examples 4-15

The following compounds were prepared according to the method of Example 3,using the appropriate amine in place of morpholine.

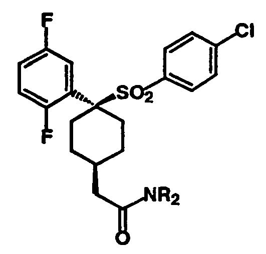

| **Ex.** | **-NR₂** | **Formula** | **M.W.** | **m/z (ES⁺) (M+1)** |
|---|---|---|---|---|
| 4 | 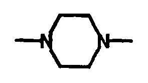 | | 510 | 511 |
| | | C₂₅H₂₉ClF₂N₂O₃S | 512 | 513 |
| 5 | 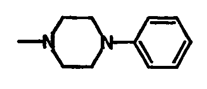 | | 572 | 573 |
| | | C₃₀H₃₁ClF₂N₂O₃S | 574 | 575 |
| 6 | 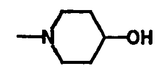 | | 511 | 512 |
| | | C₂₆H₂₈ClF₂NO₄S | 513 | 514 |
| 7 | 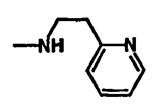 | | 532 | 533 |
| | | C₂₇H₂₇ClF₂N₂O₃S | 534 | 535 |
| 8 | 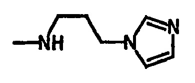 | | 535 | 536 |
| | | C₂₆H₂₈ClF₂N₃O₃S | 537 | 538 |
| 9 | 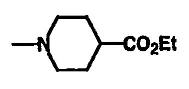 | | 567 | 568 |
| | | C₂₈H₃₂ClF₂NO₅S | 569 | 570 |
| 10 | 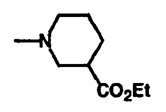 | | 567 | 568 |
| | | C₂₈H₃₂ClF₂NO₅S | 569 | 570 |
| 11 | 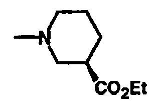 | | 567 | 568 |
| | | C₂₈H₃₂ClF₂NO₅S | 569 | 570 |
| 12 | 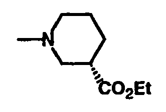 | | 567 | 568 |
| | | C₂₈H₃₂ClF₂NO₅S | 569 | 570 |
| 13 | 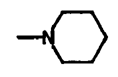 | | 495 | 496 |
| | | C₂₅H₂₈ClF₂NO₃S | 497 | 498 |
| 14 | 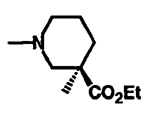 | | 581 | 582 |
| | | C₂₉H₃₄ClF₂NO₅S | 583 | 584 |
| 15 | 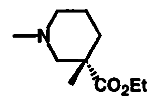 | | 581 | 582 |
| | | C₂₉H₃₄ClF₂NO₅S | 583 | 584 |

### Examples 16-33

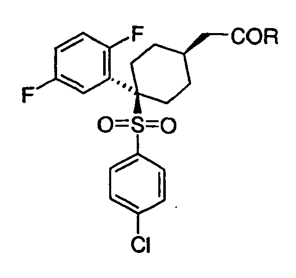

These Examples were prepared by the following method, using the appropriate amine free base or amine salt with prior neutralization.

To a stirred suspension of *cis* 4-(4-chlorobenzenesulphonyl)-4-(2,5-difluorophenyl)cyclohexaneacetic acid (Example 2, 0.15g, 0.35mmol) in dichloromethane (5ml) was added oxalyl chloride (0.05ml, 0.57mmol) and dimethylformamide (1 drop). After 30minutes the solution was evaporated to a small volume and to a solution of the residue in dichloromethane (5ml) was added the desired amine (1.75mmol). After stirring the solution for 20 minutes the solvent was removed in vacuo and the residue purified by chromatography on silica gel eluting with increasing concentrations of ethyl acetate in isohexane (25%, 50%). The fractions containing the product were evaporated to give the product amide. Chromatographic purification was performed on silica gel using appropriate concentrations of ethyl acetate in isohexane, ethyl acetate or methanol in ethyl acetate where appropriate.

| **Example No.** | **R** | **MS m/z (M+H)** | **m.p.** |
|---|---|---|---|
| 16 | NH-cyclobutyl | 482,484 | 192-193°C |
| 17 | NH₂ | 428,430 | 187-189 °C |
| 18 | NHMe | 442,444 | 200-201 °C |
| 19 | NHEt | 456,458 | 146-147 °C |
| 20 | NHⁿPr | 470,472 | 150-151 °C |
| 21 | NHⁱPr | 470,472 | 124-125 °C |
| 22 | NMe₂ | 456,458 | |
| 23 | NHCH₂CH₂Ph | 532,534 | |
| 24 | NHCH₂CF₃ | 510,512 | |
| 25 | 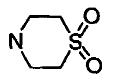 | 546,548 | |
| 26 | NHCH₂-cyclopropyl | 482,484 | 187-188 °C |
| 27 | NH-cyclopentyl | 496,498 | 182-183 °C |
| 28 | NH-cyclopropyl | 468,470 | 145-147 °C |
| 29 | NHⁿBu | 484,486 | oil |
| 30 | NH^{t}Bu | 484,486 | 102-110 °C |
| 31 | NHCH(Et)₂ | 498,500 | 89-92 °C |
| 32 | NH-allyl | 468,470 | 132-134 °C |

### Example 33

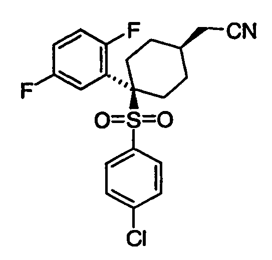

To a solution of the *cis* amide from Preparation 9 (46mg) and pyridine (0.053ml) in tetrahydrofuran (1ml) was added trifluoroacetic anhydride (0.056ml). The solution was stirred at room temperature for 2 hours when 0.5M-HCl (aqueous) and ethyl acetate were added. The organic phase was dried (MgSO₄), evaporated to a small volume and purified by chromatography on silica gel, eluting with isohexane : ethyl acetate (5 : 1) to give the desired product as a colourless solid. ¹H NMR (360MHz, CDCl₃) δ 1.61-1.70 (2H, m), 1.86-1.94 (2H, m), 2.03-2.10 (1H, m), 2.42-2.45 (4H, m), 2.51(2H, d J 8.0Hz), 6.8 (1H, m), 7.02-7.09(2H, m), 7.30 (2H, d J 8.6Hz), 7.36(2H, d J 8.7Hz).

### Example 34

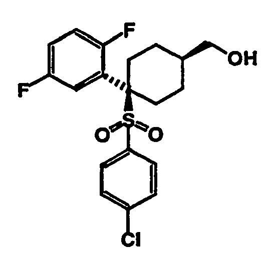

The acid from Preparation 8 (153mg) was dissolved in dry THF (10ml) and cooled to 0°C under nitrogen. Triethylamine (61µL, 0.43mmol) and isobutylchloroformate (57µL, 0.43mmol) were added and the mixture stirred at 0°C for one hour. The precipitate that had formed was removed by filtration and washed with a further 5ml of dry THF. The combined THF layers were recooled to 0°C and sodium borohydride (70mg, 1.84mmol) as a solution in water (2ml) was added with effervescence. After stirring for 30 minutes at 0°C, the reaction was diluted with ethyl acetate, washed with ammonium chloride solution, sodium bicarbonate solution and brine then dried (MgSO₄) and evaporated to dryness. The residue was purified by column chromatography eluting with ethyl acetate: hexane (1:3) to afford the desired alcohol (75mg). ¹H NMR (CDCl₃) 7.39-7.31 (4H, m), 7.10-7.01 (2H, m), 6.88-6.81 (1H, m), 3.71 (2H, d, J= 7.5Hz), 2.46-2.32 (4H, m), 1.90-1.85 (2H, m), 1.78-1.74 (1H, m) and 1.54-1.44 (2H, m). m/z =423 [MNa]⁺

### Example 35

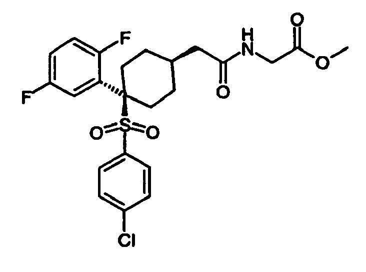

### Step (1)

To a solution of the acid from Example 2 (1 g) in DCM (50 ml) and ethyl acetate (30 ml) was added pentafluorophenol (1.5 equiv.) and DCC (1.5 equiv.) and stirred at room temperature for 1h. The reaction mixture was evaporated in vacuo, taken up in ethyl acetate and filtered. The filtrate was evaporated in vacuo to yield the pentafluorophenol ester of sufficient purity to use in subsequent reactions.

### Step (2)

To the pentafluorophenol ester prepared in Step (1) (155 mg, 0.25 mmol) dissolved in DMF (3 ml) and under nitrogen were added glycine methyl ester hydrochloride (125 mg, 1.0 mmol) and triethylamine (0.15 ml). After 2 h the reaction was diluted with water, extracted with ethyl acetate (x3), washed with, water, brine, dried (MgSO₄), filtered and evaporated. Purified by flash column chromatography (1:1 ⁱhexane/ethyl acetate to 9:1 ethyl acetate/methanol) to give a white solid (55 mg). ¹H NMR (CDCl₃) 1.08-1.16 (1H, m), 1.30-1.37 (1H, m), 1.67-1.71 (1H, m), 1.75-1.79 (2H, m), 1.91-1.95 (1H, m), 2.20-2.26 (1H, m), 2.41 (4 H, d, J= 7.8Hz), 3.77 (3H, s), 4.05 (2H, d, J= 5.1Hz), 6.19 (1H, br), 6.79-6.85 (1H, m), 7.00-7.07 (2H, m), 7.30-7.37 (4H, m).

### Example 36

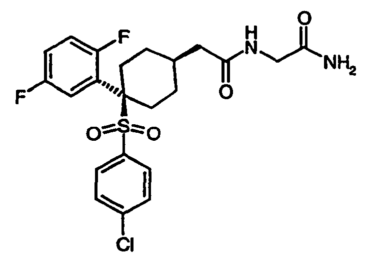

The glycine ester prepared in Example 35 (50 mg, 0.1 mmol) in a sealed tube and dissolved in a 2M ammonia in methanol solution (3 ml) was heated to 50°C for 3 h. After cooling to room temperature the reaction mixture was concentrated and purified by trituration with ether to give a white solid (28 mg). MS(EI+): 485 (MH+)

### Example 37

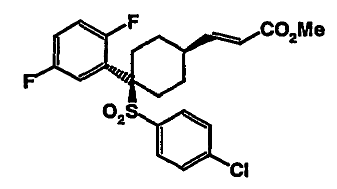

The alcohol from Example 34 (4g, 10mmol) was dissolved in dichloromethane (280ml) and was treated with Dess Martin periodinane (4.66g, 11mmol) and the mixture was stirred for 45 mins before adding saturated aqueous sodium bisulphite (100ml) and after 5mins the mixture was separated and the organic phase as washed with saturated aqueous sodium bicarbonate (100ml) dried (MgSO₄) and evaporated to dryness. The crude residue (4g) was dissolved in dry dichloromethane (100ml) and treated with methyl triphenylphosphinoacetate (4.7g 14mmol), stirring at rt. for 16hrs. The solvent was evaporated and the residue was purified by column chromatography on silica gel eluting with 10-20% ethyl acetate in hexanes, to give the product. ¹H NMR (CDCl₃) 7.37-7.36 (4H, m), 7.10-7.02 (3H, m), 6.87-6.83 (1H, m),5.91 (1H, d, J = 16Hz), 3.77 (3H, s), 2.55-2.45 (3H, m), 2.40-2.38 (2H, m), 1.95-1.90 (2H,m) and 1.65-1.52 (2H, m).

### Example 38

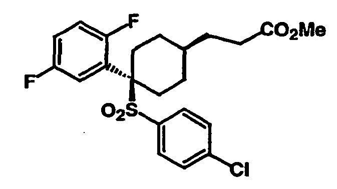

The alkene from Example 37 (3.6g, 9mmol) was dissolved in ethyl acetate (350ml). The flask was degassed and then 10% palladium on carbon (400mg) was added and the mixture stirred under an atmosphere of hydrogen for 45mins. The solution was filtered through Celite^{™} and evaporated. The clear oil obtained was purified by preparative tlc eluting with 5% ethyl acetate in hexanes. The oil obtained was then further purified by column chromatography on silica gel eluting with 5-10% ethyl acetate in hexane to give the product. ¹H NMR (CDCl₃) 7.37-7.34 (4H, m), 7.08-7.00 (2H, m), 6.85-6.81 (1H, m), 3.67 (3H, s), 2.45-2.39 (4H, m), 2.33 (2H, t, J= 8.4Hz), 1.81 (2H, q, J= 8.4Hz), 1.72-1.68 (2H,m) and 1.60-1.43 (3H, m).

### Example 39

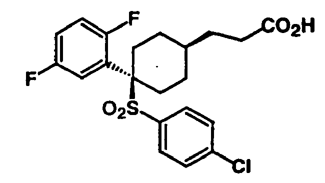

The ester from Example 38 (104mg, 0.23mmol) was dissolved in a mixture of ethanol (10ml) and water (3ml) and stirred at 20°C. The flask was degassed and then lithium hydroxide (27mg, 1.15mmol) was added. The mixture was stirred for 3hrs. at room temperature. 1N Hydrochloric acid was then added and the mixture was washed with ethyl acetate (2 x 50ml). The organic phase was washed with brine (50ml), dried (MgSO₄) and evaporated. The oil obtained was then further purified by preparative tlc eluting with ethyl acetate to give the acid. ¹H NMR (CDCl₃) 7.37-7.30 (4H, m), 7.09-6.99 (2H, m), 6.85-6.79 (1H, m), 2.42-2.36 (6H, m), 1.85-1.79 (2H, m), 1.73-1.69 (2H,m), 1.63-1.58 (1H,m) and 1.53-1.45 (2H, m).

### Example 40

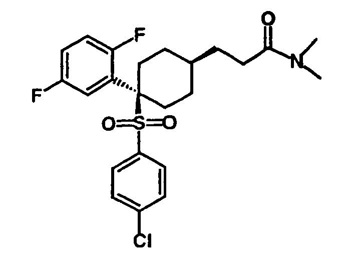

The acid from Example 39 (52 mg, 0.118 mmol) in dichloromethane (2 ml) was treated with oxalyl chloride (88µl, 2 M solution in dichloromethane, 0.176 mmol). A drop of N,N-dimethylformamide was added and the solution allowed to stir for 2 hours. After this time, solvent was removed *in vacuo* and the residue redissolved in dichloromethane (1 ml). This solution was dripped into methanolic ammonia (2 M, 2 ml). The reaction was evaporated *in vacuo* and the residue chromatographed on silica, eluting with 80% ethyl acetate in hexanes. The resulting material was purified further by preparative t.l.c., eluting with 100% ethyl acetate followed by recrystallisation from hot hexane to give product (7.4 mg, 14%). ¹H NMR (360MHz, CDCl₃), 1.45-1.53 (2H, m), 1.57-1.65 (1H, br), 1.70-1.75 (2H, m), 1.78-1.84 (2H, m), 2.32 (2H, t, J= 15.3 Hz), 2.38-2.44 (4H, br), 2.95 (3H, s), 3.02 (3H, s), 6.79-6.86 (1H, m), 7.00-7.09 (2H, m), 7.31-7.37 (4H, m); ms. (ES⁺), 470 (M⁺1), 294 (M⁺175).

### Example 41

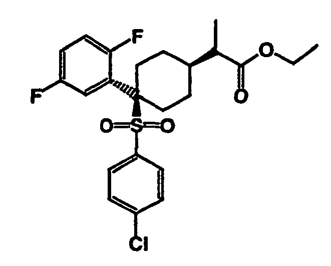

The cis-ester from Example 1 (669 mg, 1.467 mmol) in tetrahydrofuran (14 ml) was cooled to -78°C, treated with sodium bis(trimethylsilyl)amide (2.20 ml, 1 M solution in tetrahydrofuran, 2.20 mmol) and stirred while warming to room temperature over 2 hours. Methyl iodide (457 µl, 7.36 mmol) was then added to the mixture at -20°C and stirring continued, again warming to room temperature, for 2 hours. The reaction was quenched with glacial acetic acid (132 µl, 2.20 mmol), diluted with ammonium chloride (50% aq., 80 ml) and extracted with ethyl acetate (3 x 100 ml). Combined organics were then washed with brine (sat., 200 ml), dried (MgSO₄) and evaporated *in vacuo* to give crude (670 mg). This material was chromatographed on silica, eluting with 8% ethyl acetate in hexanes to give product (272 mg, 40%). ¹H NMR, (400 MHz, CDCl₃), 1.16 (3H, d, J= 6.9 Hz), 1.28 (3H, t, J= 7.1 Hz), 1.45-1.51 (2H, m), 1.71-1.77 (2H, m), 1.89-1.94 (1H, m), 2.28-2.48 (3H, br), 2.54-2.60 (1H, br), 2.70-2.74 (1H, m), 4.09-4.18 (2H, m), 6.77-6.84 (1H, m), 6.99-7.08 (2H, m), 7.26-7.36 (4H, m).

### Example 42

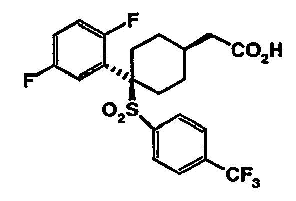

Prepared from the ketone of Preparation 3, following the procedures of Preparation 4 and Examples 1 and 2. ¹H NMR (360MHz,CDCl₃) 1.52-1.61 (2H, m), 1.76-1.81 (2H, m), 2.20-2.26 (1H, m), 2.39 (2H, d, J= 7.6Hz), 2.40-2.50 (4H, m), 5.37 (1H, br), 5.51 (1H, br), 6.75-6.83 (1H, m), 7.01-7.08 (2H, m), 7.51 (2H, d, J=8.3Hz) and 7.64 (2H, d, J= 8.3Hz).

### Example 43

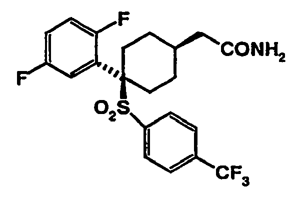

Prepared from the acid of Example 42 by the procedure of Example 35, using ammonia in the second step. MS MH+ 462(463).

### Example 44

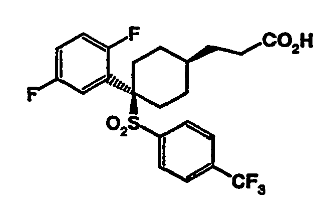

Prepared from the ketone of Preparation 3, following the procedures of Preparations 5-8 and Examples 34, 37, 38 and 39.
¹H NMR (360MHz,CDCl₃) δ 10-1 (1H, m), 7.64 (2H, d, J=8.3Hz), 7.53 (2H, d, J=8.3Hz), 7.09-7.00 (2H, m), 6.83-6.76 (1H, m), 2.50-2.37 (6H, m), 1.85-1.81 (2H, q, J=7.4Hz), 1.75-1.70 (2H, m), 1.63-1.59) (1H, m), 1.55-1.45 (2H, m).
MS(EI⁺) 477 (MH⁺).

## Claims

1. A compound of formula I: 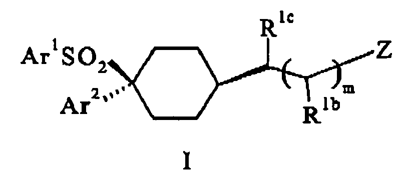 wherein:
m is 0 or 1;
Z represents CN, OR^{2a}, CO₂R^{2a} or CON(R^{2a})₂;
R^{1b} represents H, C₁₋₄alkyl or OH;
R^{1c} represents H or C₁₋₄alkyl;
Ar¹ represents phenyl or pyridyl, either of which bears 0-3 substituents independently selected from halogen, CN, NO₂, CF₃, OH, OCF₉, C₁₋₄alkoxy or C₁₋₄alkyl which optionally bears a substituent selected from halogen, CN, NO₂, CF₃, OH and C₁₋₄alkoxy;
Ar² represents phenyl which is substituted in the 2- and 5-positions with halogen;
R^{2a} represents H, C₁₋₆alkyl, C₃₋₆cycloalkyl, C₃₋₆cycloalkylC₁₋₆alkyl, C₂₋₅ alkenyl, any of which optionally bears a substituent selected from halogen, CN, NO₂, CF₃, OR^{2b}, CO₂R^{2b}, N(R^{2b})₂, CON(R^{2b})₂, Ar and COAr; or R^{2a} represents Ar; or two R^{2a} groups together with a nitrogen atom to which they are mutually attached may complete an N-heterocyclyl group bearing 0-4 substituents independently selected from =O, =S, halogen, C₁₋₄alkyl, CN, NO₃, CF₃, OH, C₁₋₄alkoxy, C₁₋₄alkoxycarbonyl, CO₂H, amino, C₁₋₄ alkylamino, di(C₁₋₄alkyl)amino, carbamoyl, Ar and COAr;
R^{2b} represents H, C₁₋₆alkyl, C₃₋₆cycloalkyl, C₃₋₆cycloalkylC₁₋₆alkyl, C₂₋₆ alkenyl, any of which optionally bears a substituent selected from halogen, CN, NO₂, CF₃, OH, C₁₋₄alkoxy, C₁₋₄alkoxycarbonyl, CO₂H, amino, C₁₋₄alkylamino, di(C₁₋₄alkyl)amino, carbamoyl, Ar and COAr; or R^{2b} represents Ar; or two R^{2b} groups together with a nitrogen atom to which they are mutually attached may complete an N-heterocyclyl group bearing 0-4 substituents independently selected from =O, =S, halogen, C₁₋₄alkyl, CN, NO₂, CF₈, OH, C₁₋₄alkoxy, C₁₋₄alkoxycarbonyl, CO₂H, amino, C₁₋₄ alkylamino, di(C₁₋₄alkyl)amino, carbamoyl, Ar and COAr;
Ar represents phenyl or heteroaryl bearing 0-3 substituents selected from halogen, C₁₋₄alkyl, CN, NO₂, CF₃, OH, C₁₋₄alkoxy, C₁₋₄alkoxycarbonyl, amino, C₁₋₄alkylamino, di(C₁₋₄alkyl)amino, carbamoyl, C₁₋₄alkylcarbamoyl and di(C₁₋₄alkyl)carbamoyl;
or a pharmaceutically acceptable salt thereof.

2. A compound according to claim 1 wherein Ar¹ is selected from phenyl groups substituted in the 4-position with halogen, methyl or trifluoromethyl, and phenyl groups substituted in the 3- and 4-positions by halogen.

3. A compound according to claim 1 or claim 2 wherein Ar¹ is 4-chlorophenyl or 4-trifluoromethylphenyl and Ar² is 2,5-difluorophenyl.

4. A compound according to any of claims 1-3 wherein Z represents CO₂R^{2a} and R^{2a} represents H or C₁₋₆alkyl.

5. The compound according to claim 1 wherein m is 1, Ar¹ represents 4-chlorophenyl, Ar² represents 2,5-difluorophenyl, R^{1b} and R^{1c} both represent H, and Z represents CO₂H, or a pharmaceutically acceptable salt thereof.

6. The compound of claim 5 in the form of its sodium salt.

7. The compound according to claim 1 wherein m is 1, Ar¹ represents 4-trifluoromethylphenyl, Ar² represents 2,5-difluorophenyl, R^{1b} and R^{1c} both represent H, and Z represents CO₂H, or a pharmaceutically acceptable salt thereof.

8. The compound according to claim 1 wherein m is 0, Ar¹ represents 4-chlorophenyl, Ar² represents 2,5-difluorophenyl, R^{1c} represents H and Z represents CONH₂, or a pharmaceutically acceptable salt thereof.

9. The compound according to claim 1 wherein m is 0, Ar¹ represents 4-trifluoromethylphenyl, Ar² represents 2,5-difluorophenyl, R^{1c} represents H and Z represents CONH₂, or a pharmaceutically acceptable salt thereof.

10. The compound according to claim 1 wherein m is 0, Ar¹ represents 4-chlorophenyl, Ar² represents 2,5-difluorophenyl, R^{1c} represents H and Z represents CONHC₂CH₃, or a pharmaceutically acceptable salt thereof.

11. The compound according to claim 1 wherein m is 0, Ar¹ represents 4-chlorophenyl, Ar² represents 2,5-difluorophenyl, R^{1c} represents H and Z represents CN, or a pharmaceutically acceptable salt thereof.

12. A pharmaceutical composition comprising a compound according to any of claims 1-11, or a pharmaceutically acceptable salt thereof, and a pharmaceutically acceptable carrier.

13. A compound according to any of claims 1-11, or a pharmaceutically acceptable salt thereof, for use in a method of treatment of the human body.

14. The use of a compound according to any of claims 1-11, or a pharmaceutically acceptable salt thereof, in the manufacture of a medicament for treatment or prevention of Alzheimer's disease.

## Patentansprüche

1. Eine Verbindung der Formel I: 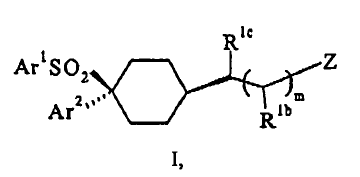 wobei:
m 0 oder 1 ist,
Z CN, OR^{2a}, CO₂R^{2a} oder CON(R^{2a})₂ bedeutet,
R^{1b} H, C₁₋₄-Alkyl oder OH bedeutet,
R^{1c} H oder C₁₋₄-Alkyl bedeutet,
Ar¹ Phenyl oder Pyridyl bedeutet, wobei jedes davon 0-3 Substituenten trägt, unabhängig ausgewählt aus Halogen, CN, NO₂, CF₃, OH, OCF₃, C₁₋₄-Alkoxy oder C₁₋₄-Alkyl, welches gegebenenfalls einen Substituenten trägt, ausgewählt aus Halogen, CN, NO₂, CF₃, OH und C₁₋₄-Alkoxy,
Ar² Phenyl bedeutet, das in den 2- und 5-Positionen mit Halogen substituiert ist,
R^{2a} H, C₁₋₆-Alkyl, C₃₋₆-Cycloalkyl, C₃₋₆-Cycloalkyl-C₁₋₆-alkyl, C₂₋₆-Alkenyl bedeutet, wobei jedes davon gegebenenfalls einen Substituenten trägt, ausgewählt aus Halogen, CN, NO₂, CF₃, OR^{2b}, CO₂R^{2b}, N(R^{2b})₂, CON(R^{2b})₂, Ar und COAr, oder R^{2a} Ar bedeutet, oder zwei R^{2a}-Gruppen zusammen mit einem Stickstoffatom, an das sie gemeinsam gebunden sind, eine N-Heterocyclylgruppe vervollständigen können, die 0-4 Substituenten trägt, unabhängig ausgewählt aus =O, =S, Halogen, C₁₋₄-Alkyl, CN, NO₂, CF₃, OH, C₁₋₄-Alkoxy, C₁₋₄-Alkoxycarbonyl, CO₂H, Amino, C₁₋₄-Alkylamino, Di(C₁₋₄-alkyl)amino, Carbamoyl, Ar und COAr,
R^{2b} H, C₁₋₆-Alkyl, C₃₋₆-Cycloalkyl, C₃₋₆-cycloalkyl-C₁₋₆-alkyl, C₂₋₆-Alkenyl bedeutet, wobei jedes davon gegebenenfalls einen Substituenten trägt, ausgewählt aus Halogen, CN, NO₂, CF₃, OH, C₁₋₄-Alkoxy, C₁₋₄-Alkoxycarbonyl, CO₂H, Amino, C₁₋₄-Alkylamino, Di(C₁₋₄-alkyl)amino, Carbamoyl, Ar und COAr, oder R^{2b} Ar bedeutet, oder zwei R^{2b}-Gruppen zusammen mit einem Stickstoffatom, an das sie gemeinsam gebunden sind, eine N-Heterocyclylgruppe vervollständigen können, die 0-4 Substituenten trägt, unabhängig ausgewählt aus =O, =S, Halogen, C₁₋₄-Alkyl, CN, NO₂, CF₃, OH, C₁₋₄-Alkoxy, C₁₋₄-Alkoxycarbonyl, CO₂H, Amino, C₁₋₄-Alkylamino, Di(C₁₋₄-alkyl)amino, Carbamoyl, Ar und COAr,
Ar Phenyl oder Heteroaryl bedeutet, das 0-3 Substituenten trägt, ausgewählt aus Halogen, C₁₋₄-Alkyl, CN, NO₂, CF₃, OH, C₁₋₄-Alkoxy, C₁₋₄-Alkoxycarbonyl, Amino, C₁₋₄-Alkylamino, Di(C₁₋₄-alkyl)amino, Carbamoyl, C₁₋₄-Alkylcarbamoyl und Di(C₁₋₄-alkyl)carbamoyl,
oder ein pharmazeutisch annehmbares Salz davon.

2. Eine Verbindung gemäß Anspruch 1, wobei Ar¹ ausgewählt ist aus Phenylgruppen, substituiert in der 4-Position mit Halogen, Methyl oder Trifluormethyl, und Phenylgruppen, substituiert in den 3- und 4-Positionen durch Halogen.

3. Eine Verbindung gemäß Anspruch 1 oder Anspruch 2, wobei Ar¹ 4-Chlorphenyl oder 4-Trifluormethylphenyl ist und Ar² 2,5-Difluorphenyl ist.

4. Eine Verbindung gemäß irgendeinem der Ansprüche 1-3, wobei Z CO₂R^{2a} bedeutet und R^{2a} H oder C₁₋₆-Alkyl bedeutet.

5. Die Verbindung gemäß Anspruch 1, wobei m 1 ist, Ar¹ 4-Chlorphenyl bedeutet, Ar² 2,5-Difluorphenyl bedeutet, R^{1b} und R^{1c} beide H bedeuten und Z CO₂H bedeutet, oder ein pharmazeutisch annehmbares Salz davon.

6. Die Verbindung nach Anspruch 5 in Form ihres Natriumsalzes.

7. Die Verbindung gemäß Anspruch 1, wobei m 1 ist, Ar¹ 4-Trifluormethylphenyl bedeutet, Ar² 2,5-Difluorphenyl bedeutet, R^{1b} und R^{1c} beide H bedeuten und Z CO₂H bedeutet, oder ein pharmazeutisch annehmbares Salz davon.

8. Die Verbindung gemäß Anspruch 1, wobei m 0 ist, Ar¹ 4-Chlorphenyl bedeutet, Ar² 2,5-Difluorphenyl bedeutet, R^{1c} H bedeutet und Z CONH₂ bedeutet, oder ein pharmazeutisch annehmbares Salz davon.

9. Die Verbindung gemäß Anspruch 1, wobei m 0 ist, Ar¹ 4-Trifluormethylphenyl bedeutet, Ar² 2,5-Difluorphenyl bedeutet, R^{1c} H bedeutet und Z CONH₂ bedeutet, oder ein pharmazeutisch annehmbares Salz davon.

10. Die Verbindung gemäß Anspruch 1, wobei m 0 ist, Ar¹ 4-Chlorphenyl bedeutet, Ar² 2,5-Difluorphenyl bedeutet, R^{1c} H bedeutet und Z CONHCH₂CH₃ bedeutet, oder ein pharmazeutisch annehmbares Salz davon.

11. Die Verbindung gemäß Anspruch 1, wobei m 0 ist, Ar¹ 4-Chlorphenyl bedeutet, Ar² 2,5-Difluorphenyl bedeutet, R^{1c} H bedeutet und Z CN bedeutet, oder ein pharmazeutisch annehmbares Salz davon.

12. Eine pharmazeutische Zusammensetzung, die eine Verbindung gemäß irgendeinem der Ansprüche 1-11 oder ein pharmazeutisch annehmbares Salz davon und einen pharmazeutisch annehmbaren Träger enthält.

13. Eine Verbindung gemäß irgendeinem der Ansprüche 1-11 oder ein pharmazeutisch annehmbares Salz davon zur Verwendung bei einem Verfahren zur Behandlung des menschlichen Körpers.

14. Die Verwendung einer Verbindung gemäß irgendeinem der Ansprüche 1-11 oder eines pharmazeutisch annehmbaren Salzes davon bei der Herstellung eines Medikaments zur Behandlung oder Prävention von Alzheimer-Krankheit.

## Revendications

1. Composé de formule I : 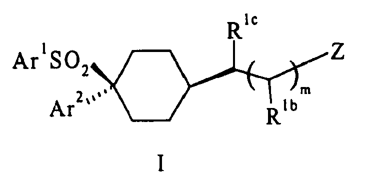 dans laquelle
m vaut 0 ou 1 ;
Z représente CN, OR^{2a}, CO₂R^{2a} ou CON(R^{2a})₂ ;
R^{1b} représente H, un groupe alkyle en C₁-C₄ ou OH ;
R^{1c} représente H ou un groupe alkyle en C₁-C₄ ;
Ar¹ représente un groupe phényle ou pyridyle, portant de 0 à 3 substituants indépendamment choisis parmi un atome d'halogène, un groupe CN, NO₂, CF₃, OH, OCF₃, alcoxy en C₁-C₄ ou alkyle en C₁-C₄ qui porte éventuellement un substituant choisi parmi un atome d'halogène, les groupes CN, NO₂, CF₃, OH et alcoxy en C₁-C₄ ;
Ar² représente un groupe phényle qui est substitué en positions 2 et 5 par un atome d'halogène ;
R^{2a} représente H, les groupes alkyle en C₁-C₆, cycloalkyle en C₃-C₆, (cycloalkyle en C₃-C₆) (alkyle en C₁-C₆), alcényle en C₂-C₆, qui portent éventuellement un substituant choisi parmi un atome d'halogène, les groupes CN, NO₂, CF₃, OR^{2b}, CO₂R^{2b}, N(R^{2b})₂, CON(R^{2b})₂, Ar et COAr ; ou
R^{2a} représente Ar, ou deux groupes R^{2a} ensemble avec l'atome d'azote auquel ils sont mutuellement attachés peuvent former un groupe N-hétérocyclyle portant de 0 à 4 substituants indépendamment choisis parmi =O, =S, un atome d'halogène, les groupes alkyle en C₁-C₄, CN, NO₂, CF₃, OH, alcoxy en C₁-C₄, (alcoxy en C₁-C₄) carbonyle, CO₂H, amino, (alkyle en C₁-C₄) amino, di(alkyle en C₁-C₄)amino, carbamoyle, Ar, et COAr ;
R^{2b} représente H, les groupes alkyle en C₁-C₆, cycloalkyle en C₃-C₆, (cycloalkyle en C₃-C₆) (alkyle en C₁-C₆), alcényle en C₂-C₆, qui portent éventuellement un substituant choisi parmi un atome d'halogène, les groupes CN, NO₂, CF₃, OH, alcoxy en C₁-C₄, (alcoxy en C₁-C₄) carbonyle, CO₂H, amino, (alkyle en C₁-C₄) amino, di(alkyle en C₁-C₄)amino, carbamoyle, Ar, et COAr ; ou R^{2b} représente Ar, ou deux groupes R^{2b} ensemble avec l'atome d'azote auquel ils sont mutuellement attachés peuvent former un groupe N-hétérocyclyle portant de 0 à 4 substituants indépendamment choisis parmi =O, =S, un atome d'halogène, les groupes alkyle en C₁-C₄, CN, NO₂, CF₃, OH, alcoxy en C₁-C₄, (alcoxy en C₁-C₄)carbonyle, CO₂H, amino, (alkyle en C₁-C₄)amino, di(alkyle en C₁-C₄)amino, carbamoyle, Ar, et COAr ;
Ar représente un groupe phényle ou hétéroaryle portant de 0 à 3 substituants choisis parmi un atome d'halogène, les groupe alkyle en C₁-C₄, CN, NO₂, CF₃, OH, alcoxy en C₁-C₄, (alcoxy en C₁-C₄) carbonyle, amino, (alkyle en C₁-C₄)amino, di(alkyle en C₁-C₄)amino, carbamoyle, (alkyle en C₁-C₄)carbamoyle, et di(alkyle en C₁-C₄)carbamoyle ;
ou un sel pharmaceutiquement acceptable de celui-ci.

2. Composé selon la revendication 1, dans lequel Ar¹ est choisi parmi les groupes phényles substitués en position 4 par un atome d'halogène, un groupe méthyle ou trifluorométhyle, et les groupes phényles substitués en positions 3 et 4 par un atome d'halogène.

3. Composé selon la revendication 1 ou la revendication 2, dans lequel Ar¹ est un groupe 4-chlorophényle ou 4-trifluorométhylphényle et Ar² est un groupe 2,5-difluorophényle.

4. Composé selon l'une quelconque des revendications 1 à 3, dans lequel Z représente CO₂R^{2a}, et R^{2a} représente H ou un groupe alkyle en C₁-C₆.

5. Composé selon la revendication 1, dans lequel m vaut 1, Ar¹ représente un groupe 4-chlorophényle, Ar² représente un groupe 2,5-difluorophényle, R^{1b} et R^{1c} représentent tous les deux H, et Z représente CO₂H, ou un sel pharmaceutiquement acceptable de celui-ci.

6. Composé selon la revendication 5, sous la forme de son sel sodique.

7. Composé selon la revendication 1, dans lequel m vaut 1, Ar¹ représente un groupe 4-trifluorométhylphényle, Ar² représente un groupe 2,5-difluorophényle, R^{1b} et R^{1c} représentent tous les deux H, et Z représente CO₂H, ou un sel pharmaceutiquement acceptable de celui-ci.

8. Composé selon la revendication 1, dans lequel m vaut 0, Ar¹ représente un groupe 4-chlorophényle, Ar² représente un groupe 2,5-difluorophényle, R^{1c} représente H, et Z représente CONH₂, ou un sel pharmaceutiquement acceptable de celui-ci.

9. Composé selon la revendication 1, dans lequel m vaut 0, Ar¹ représente un groupe 4-trifluorométhylphényle, Ar² représente un groupe 2,5-difluorophényle, R^{1c} représente H, et Z représente CONH₂, ou un sel pharmaceutiquement acceptable de celui-ci.

10. Composé selon la revendication 1, dans lequel m vaut 0, Ar¹ représente un groupe 4-chlorophényle, Ar² représente un groupe 2,5-difluorophényle, R^{1c} représente H, et Z représente CONHCH₂CH₃, ou un sel pharmaceutiquement acceptable de celui-ci.

11. Composé selon la revendication 1, dans lequel m vaut 0, Ar¹ représente un groupe 4-chlorophényle, Ar² représente un groupe 2,5-difluorophényle, R^{1c} représente H, et Z représente CN, ou un sel pharmaceutiquement acceptable de celui-ci.

12. Composition pharmaceutique comprenant un composé selon l'une quelconque des revendications 1 à 11, ou un sel pharmaceutiquement acceptable de celui-ci, et un support pharmaceutiquement acceptable.

13. Composé selon l'une quelconque des revendications 1 à 11, ou sel pharmaceutiquement acceptable de celui-ci, pour une utilisation dans un procédé de traitement du corps humain.

14. Utilisation d'un composé selon l'une quelconque des revendications 1 à 11, ou d'un sel pharmaceutiquement acceptable de celui-ci, pour la fabrication d'un médicament destiné au traitement ou à la prévention de la maladie d'Alzheimer.
